# EUROPEAN PATENT APPLICATION

(11) **EP 4 825 388 A1**
(43) Date of publication of application: **21.10.2026**
(21) Application number: 24904003.1
(22) Date of filing: 12.11.2024
(51) Int. Cl.: A61K 38/05, A61P 43/00, A61P 21/00, A23L 33/18, A23K 20/147

(54) **USE OF CYCLO(HIS-PRO) (CHP) FOR ENHANCING EXERCISE PERFORMANCE, IMPROVING MUSCLE FUNCTION, OR PREVENTING, ALLEVIATING, OR TREATING MUSCLE DISEASES**

(30) Priority: 15.12.2023 KR 20230183097
(71) Applicant: Novmetapharma Co., Ltd., Seoul 06050 (KR)
(72) Inventor: JUNG, Hoe Yune, Pohang-si, Gyeongsangbuk-do 37668 (KR); AUWERX, Johan, 1015 Lausanne (CH); LEE, Do Hyun, Pohang-si, Gyeongsangbuk-do 37667 (KR); DE MASI, Alessia, 1015 Lausanne (CH); JEON, Jongsu, Pohang-si, Gyeongsangbuk-do 37664 (KR)
(74) Representative: Maiwald GmbH
(86) International application number: PCT/KR2024/017882
(87) International publication number: WO 2025/127437

(57) **Abstract**

The present invention relates to uses of cyclo(his-pro) (CHP) for enhancing exercise performance, improving muscle function, or preventing, alleviating, or treating muscle diseases and, more specifically, provides a pharmaceutical composition, a food composition, a feed additive, a method for enhancing exercise performance, a method for improving muscle function or for preventing, alleviating, or treating muscle diseases, and a method for enhancing muscle strength, each using cyclo(his-pro) as an active ingredient, wherein the cyclo(his-pro) can be used not only in enhancing exercise performance and strengthening muscle strength by exhibiting effects of increasing muscle strength, movement coordination, resistance to muscle fatigue, sense of balance, and muscle energy production, but also in enhancing muscle function or preventing, alleviating, or treating muscle diseases by exhibiting effects of increasing muscle mass and muscle fiber size, inhibiting muscle loss, muscle atrophy, and improving muscle energy production.

## Description

### TECHNICAL FIELD

This application claims priority to and the benefit of Korean Patent Application No. 10-2023-0183097, filed on December 15, 2023, the disclosure of which is incorporated herein by reference in its entirety.

The present invention relates to the use of cyclo(his-pro) (CHP) for enhancing exercise performance, improving muscle function, or preventing, ameliorating or treating a muscle disease, and more specifically, the present invention provides a pharmaceutical composition, a food composition, a feed additive, a method for enhancing exercise performance, a method for improving muscle function or for preventing, ameliorating or treating a muscle disease, and a method for enhancing muscle strength, each using cyclo(his-pro) as an active ingredient, wherein the cyclo(his-pro) can be used not only in enhancing exercise performance and strengthening muscle strength by exhibiting effects of increasing muscle strength, movement coordination, resistance to muscle fatigue, sense of balance and muscle energy production, but also in enhancing muscle function or preventing, ameliorating or treating a muscle disease by exhibiting effects of increasing muscle mass and muscle fiber size, inhibiting muscle loss, muscle atrophy and improving muscle energy production.

### BACKGROUND ART

Muscle atrophy is a progressive decrease in muscle mass, which causes muscle weakness and degeneration. Muscle atrophy is accelerated by inactivity, oxidative stress or chronic inflammation, and weakens muscle function and motor skills.

One of the representative methods for enhancing muscle function by promoting energy consumption is to increase fatty acid oxidation by mitochondria to generate ATP energy. The number and ability of mitochondria that control this are regulated by the coactivator PGC-1α (peroxisome proliferator-activated receptor-gamma coactivator 1 alpha), and it was revealed that PGC-1α activity is regulated by SIRT1 (sirtuin 1) (Non-Patent Document 1).

When the transcription factor Fox (forhead box) moves from the cytoplasm into the nucleus, it increases the expressions of the E3 ubiquitin ligase factors Atrogin-1/MAFbx (muscle atrophy F-box) and MuRF-1 (muscle RINGfinger protein-1), which are involved in protein degradation (Non-Patent Document 2). When the expression levels of these factors increase, protein degradation within the muscle is promoted, resulting in a decrease in muscle mass. Therefore, suppression of the expressions of Atrogin-1/MAFbx and MuRF-1 reduces the loss of muscle protein mass, thereby maintaining normal muscle function.

As the elderly population rapidly increases, research investments are being focused on developing treatments for geriatric diseases. In 2017, the World Health Organization (WHO) created sarcopenia related to skeletal muscle atrophy as a disease code (ICD-10-CM), and treatment development therefor is actively underway. However, to date, there are no drugs approved by the US FDA for the treatment of sarcopenia.

Meanwhile, cyclo(his-pro) (CHP) is a naturally occurring circular dipeptide composed of histidine-proline, which is a metabolite of thyrotropin-releasing hormone (TRH), or a physiologically active dipeptide that is synthesized in the body through TRH metabolism and *de novo*, and it refers to a substance that is widely distributed throughout the brain, spinal cord and gastrointestinal tract.

CHP has been reported to ameliorate diabetes and to be effective in treating neurodegenerative diseases such as Alzheimer's. In addition, CHP has been reported to be effective in treating bone loss disease (Patent Document 1), but its effects in improving muscle function, enhancing exercise performance and treating muscle diseases are known.

### [Related Art Documents]

### [Patent Documents]

(Patent Document 1) Korean Patent Laid-Open Publication Patent No. 10-2019-0058346

### [Non-Patent Documents]

(Non-Patent Document 1) Gerhart-Hines Z, Rodgers JT, Bare O, Lerin C, Kim SH, Mostoslavsky R, Alt FW, Wu Z, Puigserver P. Metabolic control of muscle mitochondrial function and fatty acid oxidation through SIRT1/PGC-1alpha. EMBO J. 2007 Apr 4;26(7):1913-23.
(Non-Patent Document 2) Bonaldo P, Sandri M. Cellular and molecular mechanisms of muscle atrophy. Dis Model Mech. 2013 Jan;6(1):25-39.

### DISCLOSURE

### TECHNICAL PROBLEM

An object of the present invention is to provide a composition for enhancing exercise performance, including cyclo(his-pro) as an active ingredient.

Another object of the present invention is to provide a composition for improving muscle function or preventing, ameliorating or treating a muscle disease, including cyclo(his-pro) as an active ingredient.

Still another object of the present invention is to provide a composition for strengthening muscle strength, including cyclo(his-pro) as an effective ingredient.

Still another object of the present invention is to provide a method for enhancing exercise performance, a method for improving muscle function, a method for preventing, ameliorating or treating a muscle disease and a method for strengthening muscle strength, by using cyclo(his-pro).

Still another object of the present invention is to provide the use of cyclo(his-pro) for the manufacture of a medicament or health functional food for enhancing exercise performance, improving muscle function, preventing, ameliorating or treating a muscle disease and strengthening muscle strength.

### TECHNICAL SOLUTION

In order to solve the above-described problems, the present invention provides a pharmaceutical composition for enhancing exercise performance, including cyclo(his-pro) or a pharmaceutically acceptable salt thereof.

In addition, the present invention provides a health functional food composition for enhancing exercise performance, including cyclo(his-pro) or a sitologically acceptable salt thereof.

In addition, the present invention provides a feed additive for enhancing exercise performance, including cyclo(his-pro) or a salt thereof.

In addition, the present invention provides a method for enhancing exercise performance, including administering an effective amount of cyclo(his-pro) or a salt thereof to a subject in need thereof.

In addition, the present invention provides the use of cyclo(his-pro) or a salt thereof for the manufacture of a medicament or health functional food for enhancing exercise performance.

In the present invention, the pharmaceutical composition may exhibit any one or more of the following effects of i) to v):
i) increased muscle strength;
ii) increased motor coordination;
iv) increased balance; and
v) increased muscle energy production.

Additionally, the present invention provides a pharmaceutical composition for improving muscle function or preventing or treating a muscle disease, including cyclo(his-pro) or a pharmaceutically acceptable salt thereof.

In addition, the present invention provides a health functional food composition for improving muscle function or preventing or ameliorating a muscle disease, including cyclo(his-pro) or a sitologically acceptable salt thereof.

In addition, the present invention provides a feed additive for improving muscle function or preventing or ameliorating a muscle disease, including cyclo(his-pro) or a salt thereof.

In addition, the present invention provides a method for improving muscle function or preventing, ameliorating or treating a muscle disease, including administering an effective amount of cyclo(his-pro) or a salt thereof to a subject in need thereof.

In addition, the present invention provides the use of cyclo(his-pro) or a salt thereof for the manufacture of a medicament or health functional food for improving muscle function or preventing, ameliorating or treating a muscle disease.

In the present invention, the muscle disease may be a muscle disease caused by muscle dysfunction, muscle atrophy, muscle loss or muscle degeneration.

In the present invention, the muscle disease may be any one or more selected from the group consisting of atony, muscular atrophy, muscular dystrophy, myasthenia, cachexia, sarcopenia, myocardia and acardiotrophy.

In the present invention, the cyclo(his-pro) or a salt thereof may exhibit any one or more of the following effects of i) to v):
i) increased muscle strength;
ii) increased motor coordination;
iii) increased tolerance to muscle fatigue;
iv) increased balance; and
v) increased muscle energy production.

Furthermore, the present invention provides a composition for strengthening muscle strength, including cyclo(his-pro) or a pharmaceutically or sitologically acceptable salt thereof.

In addition, the present invention provides a method for strengthening muscle strength, including administering an effective amount of cyclo(his-pro) or a salt thereof to a subject in need thereof.

In addition, the present invention provides the use of cyclo(his-pro) or a salt thereof for the manufacture of a medicament or health functional food for strengthening muscle strength.

### ADVANTAGEOUS EFFECTS

The composition including cyclo(his-pro) of the present invention exhibits the effects of increasing muscle strength, increasing movement coordination, increasing resistance to muscle fatigue, increasing a sense of balance and increasing muscle energy production, and thus can be utilized as a medicine, health functional food or feed additive for enhancing exercise performance or strengthening muscle strength. In addition, the composition exhibits the effects of increasing muscle mass, increasing muscle fiber size, inhibiting muscle atrophy, inhibiting muscle loss and increasing muscle energy production, and thus can be utilized as a medicine, health functional food or feed additive for improving muscle function or preventing, ameliorating or treating various muscle diseases.

### DESCRIPTION OF DRAWINGS

FIG. 1 shows the survival rate of mice in each group during the experimental period.
FIGS. 2A to 2C show the results of confirming the effects of CHP administration on improving muscle function and enhancing exercise performance in an aged animal model. FIG. 2A shows the results of grip strength measurement, FIG. 2B shows the results of a limb hanging test, and FIG. 2C shows the results of a sensorimotor function evaluation.
FIG. 3A shows the changes in the weight of gastrocnemius (GM), tibialis anterior (TA) and cardiac muscle tissues following CHP administration in aged animals.
FIGS. 3B to 3D show changes in the cross-sectional area of muscle fibers according to CHP administration in an aged animal model. FIG. 3B is a photograph of a cross-section of muscle fibers from GM muscles stained with H&E, FIG. 3C shows the average cross-sectional area of muscle fibers measured, and FIG. 3D shows the distribution of muscle fibers by cross-sectional area size as a percentage.
FIG. 4 shows the changes in gene expression in GM and TA muscles following CHP administration in an aged animal model.
FIG. 5 shows the change in citrate synthase activity in TA muscle following CHP administration in an aged animal model.
FIG. 6 shows changes in gene expression related to cardiac atrophy following CHP administration in an aged animal model.
FIG. 7A shows the results of grip strength measurements according to the CHP prevention protocol in the mdx animal model.
FIG. 7B shows the results of grip strength measurements according to the CHP treatment protocol in the mdx animal model.
FIG. 8 shows the results of the hanging test according to the CHP treatment protocol in the mdx animal model.

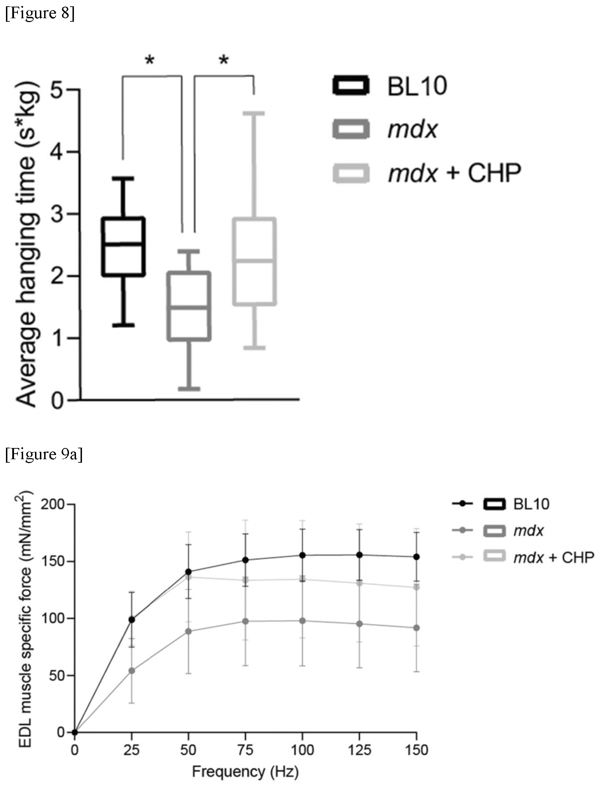

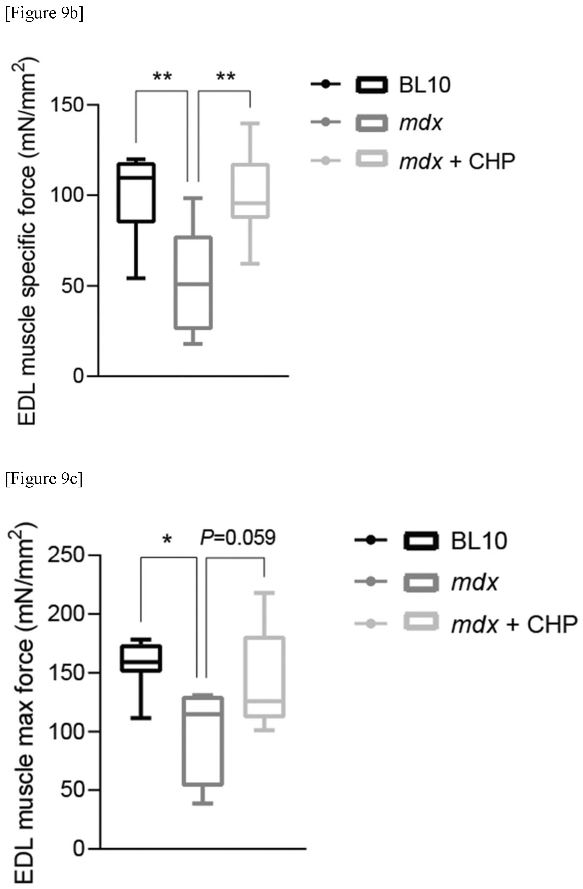

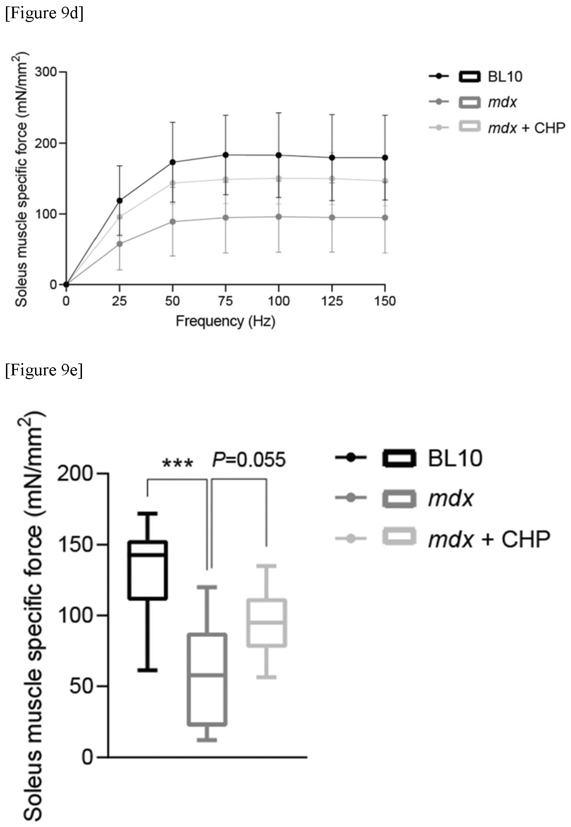

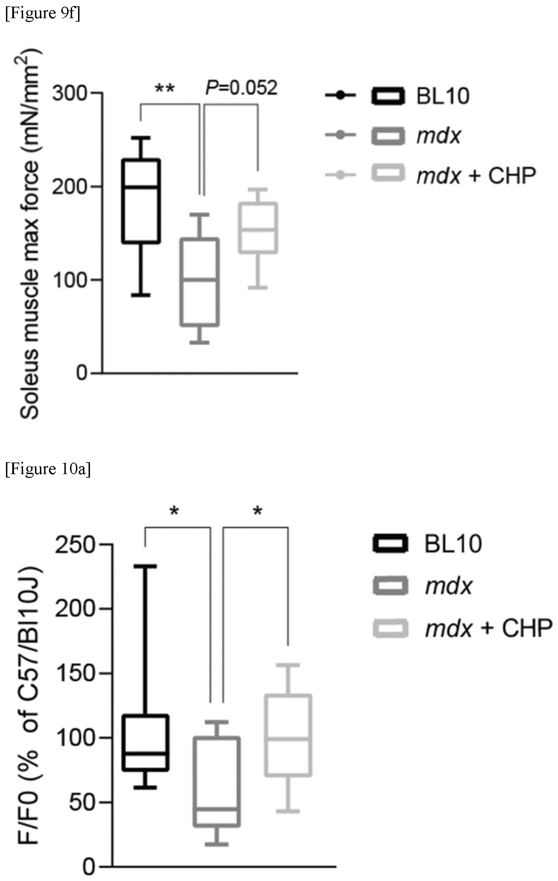

FIGS. 9A to 9F show the results of confirming the effect of maintaining force generation according to the CHP prevention protocol in the mdx animal model. FIG. 9A shows the force-frequency relationship of the extensor digitorum longus (EDL), FIG. 9B shows the maximum specific isometric force of the EDL developed by 25 Hz stimulation, FIG. 9C shows the maximum specific isometric force of the EDL developed during the test, FIG. 9D shows the force-frequency relationship of the soleus muscles, FIG. 9E shows the maximum specific isometric force of the soleus muscle developed by 25 Hz stimulation, and FIG. 9F shows the maximum specific isometric force of the soleus muscle developed during the test.

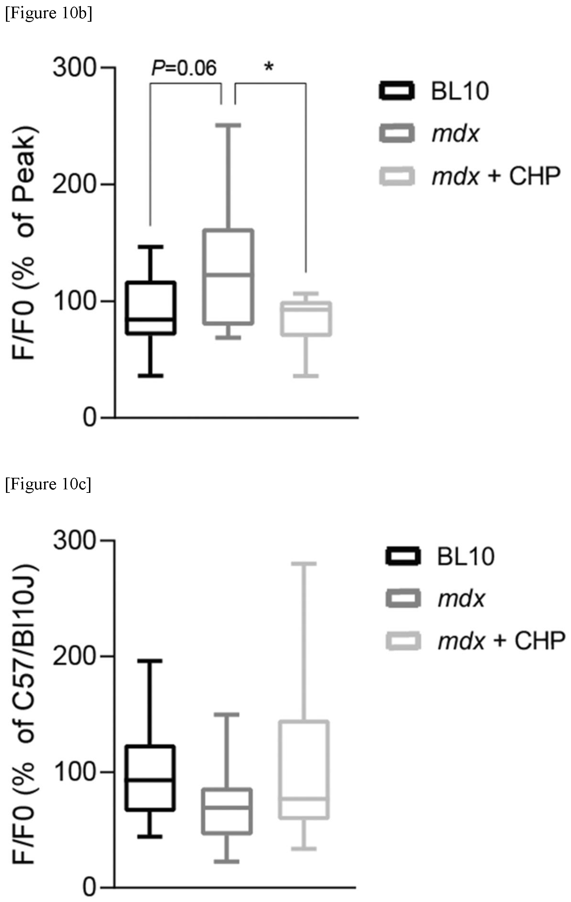

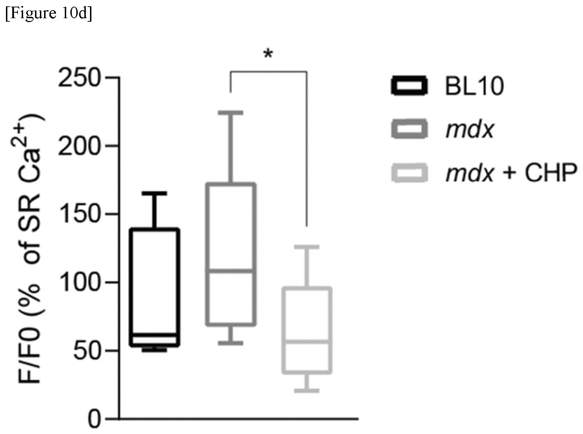

FIGS. 10A to 10D show the effects of CHP on Ca²⁺ dysregulation according to the CHP prevention protocol in the mdx animal model. FIG. 10A shows Ca²⁺ amplitude (SR storage) as a percentage of response in fibers isolated from BL10 mice upon 2.5 mM caffeine stimulation (n = 10 to 12), FIG. 10B shows SR Ca²⁺ uptake as a percentage of Ca²⁺ peak after 2.5 mM caffeine stimulation (n = 10 to 12), FIG. 10C shows Ca²⁺ amplitude (SR storage) as a percentage of response in fibers isolated from BL10 mice upon 1 µM thapsigargin stimulation (n = 6 to 14), and FIG. 10D shows SR Ca²⁺ uptake as a percentage of Ca²⁺ peak after 1 µM thapsigargin stimulation (n = 6 to 14).

### BEST MODE

Hereinafter, the present invention will be described in more detail.

As described above, with the rapid increase in the elderly population, the active development of treatments for sarcopenia related to skeletal muscle atrophy is underway, but to date, there are no drugs that have shown a clear therapeutic effect.

Accordingly, the inventors of the present invention completed the present invention by confirming that cyclo(his-pro) not only significantly enhances muscle strength, motor coordination, resistance to muscle fatigue, sense of balance and muscle energy production, but also suppresses the expressions of various genes related to aging and muscle atrophy and promotes the expressions of genes related to anti-aging and anti-oxidation including mitochondrial function, thereby exhibiting excellent effects in enhancing exercise performance, strengthening muscle strength, improving muscle function, and preventing, ameliorating or treating muscle diseases.

Accordingly, a first aspect of the present invention relates to a composition for enhancing exercise performance, comprising cyclo(his-pro) or a salt thereof, consisting essentially of the cyclo(his-pro) or a salt thereof, or consisting of the cyclo(his-pro) or a salt thereof.

Specifically, the composition may be a pharmaceutical composition for enhancing exercise performance, comprising cyclo(his-pro) or a pharmaceutically acceptable salt thereof, consisting essentially of the cyclo(his-pro) or a pharmaceutically acceptable salt thereof, or consisting of the cyclo(his-pro) or a pharmaceutically acceptable salt thereof; a health functional food composition for enhancing exercise performance, comprising cyclo(his-pro) or a sitologically acceptable salt thereof, consisting essentially of the cyclo(his-pro) or a sitologically acceptable salt thereof, or consisting of the cyclo(his-pro) or a sitologically salt thereof; or a feed additive for enhancing exercise performance, comprising cyclo(his-pro) or a salt thereof, consisting essentially of the cyclo(his-pro) or a salt thereof, or consisting of the cyclo(his-pro) or a salt thereof.

In relation to the first aspect, the present invention provides a method for enhancing exercise performance, including administering an effective amount of cyclo(his-pro) or a pharmaceutically or sitologically acceptable salt thereof to a subject in need thereof.

In relation to the first aspect, the present invention also provides the use of cyclo(his-pro) or a pharmaceutically or sitologically acceptable salt thereof for enhancing exercise performance.

In relation to the first aspect, the present invention also provides the use of cyclo(his-pro) or a pharmaceutically or sitologically acceptable salt thereof for the manufacture of a medicament or health functional food for enhancing exercise performance.

In the present invention, the cyclo(his-pro) may be used by synthesizing or as a commercially available product. In addition, it may be used by purifying from a substance containing cyclo(his-pro), such as a prostate extract or soybean hydrolysate.

By the use of the term "purified", it is meant that cyclo(his-pro) is in a concentrated form compared to the form in which it can be obtained from natural sources, such as prostate extract. Purified ingredients may be concentrated from the natural sources thereof, or obtained through chemical synthesis methods.

In the present invention, the term "exercise performance" means the ability to perform physical movements performed in daily life or sports quickly, strongly, for a long period of time, and skillfully. Exercise performance is defined by factors such as muscle strength, sense of balance, motor coordination, agility and endurance. In addition, enhancing exercise performance in the present invention is considered to be a beneficial physiological effect even in people performing daily physical activities that are not related to exercise.

The term "muscle fatigue" in the present invention means a state in which the ability to perform physical activity is temporarily reduced after intense exercise or due to long-term exercise, and is accompanied by a decrease in muscle contractility and the like. Muscle fatigue may manifest itself as symptoms of fatigue, decreased endurance, decreased explosiveness or lethargy.

In the present invention, the term "endurance" is defined as resistance to fatigue. It means resistance to fatigue that occurs during submaximal (before exerting maximum effort) sustained exercise or intense exercise. Endurance exercise usually lasts for 30 minutes or more. In particular, exercise that lasts 4 to 5 hours or more is also called ultra-endurance exercise. Additionally, in the present invention, the increase in endurance is considered to be a beneficial physiological function when performing exercise that is not limited in time (such as recreational running, walking, swimming, cycling, gymnastic training, etc.).

In a specific embodiment of the present invention, the effect of cyclo(his-pro) on enhancing exercise performance was evaluated by performing grip strength measurement, limb hanging test and sensorimotor function evaluation on an aged animal model treated with cyclo(his-pro).

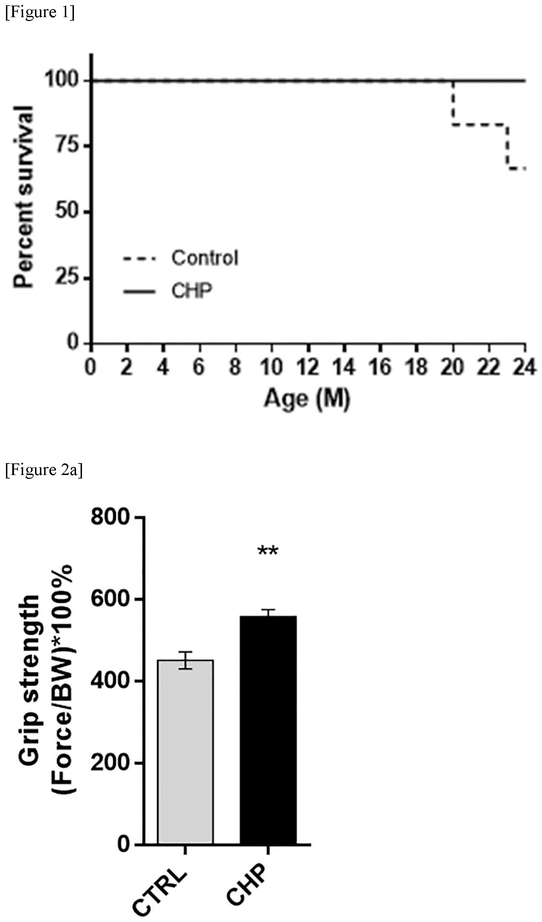

As a result, as shown in FIGS. 2A to 2C, the cyclo(his-pro) administration group significantly increased maximum muscle strength and hanging time, and significantly improved sensorimotor functions compared to the vehicle-treated control group, indicating effects of enhancing muscle strength, motor coordination, tolerance to muscle fatigue and sense of balance. Accordingly, the cyclo(his-pro) according to the present invention may be utilized in various ways as a medicine, health functional food, feed additive and the like for the purpose of enhancing exercise performance.

In addition, cyclo(his-pro) or a salt thereof according to the present invention may be administered to a subject for the purpose of enhancing exercise performance. Accordingly, provided is a method for enhancing exercise performance, including administering cyclo(his-pro) or a salt thereof to a subject in need thereof.

A second aspect of the present invention relates to a composition for improving muscle function or preventing, ameliorating or treating muscle disease, comprising cyclo(his-pro) or a salt thereof, consisting essentially of the cyclo(his-pro) or a salt thereof, or consisting of the cyclo(his-pro) or a salt thereof.

Specifically, the composition may be a pharmaceutical composition for improving muscle function or preventing or treating a muscle disease, comprising cyclo(his-pro) or a pharmaceutically acceptable salt thereof, consisting essentially of the cyclo(his-pro) or a pharmaceutically acceptable salt thereof, or consisting of the cyclo(his-pro) or a pharmaceutically acceptable salt thereof; a health functional food composition for improving muscle function or preventing or ameliorating a muscle disease, comprising cyclo(his-pro) or a sitologically acceptable salt thereof, consisting essentially of the cyclo(his-pro) or a sitologically acceptable salt thereof, or consisting of the cyclo(his-pro) or a sitologically salt thereof; or a feed additive for improving muscle function or preventing or ameliorating a muscle disease, comprising cyclo(his-pro) or a salt thereof, consisting essentially of the cyclo(his-pro) or a salt thereof, or consisting of the cyclo(his-pro) or a salt thereof.

In relation to the second aspect, the present invention provides a method for improving muscle function or preventing, ameliorating or treating a muscle disease, including administering an effective amount of cyclo(his-pro) or a pharmaceutically or sitologically acceptable salt thereof to a subject in need thereof.

In relation to the second aspect, the present invention also provides the use of cyclo(his-pro) or a pharmaceutically or sitologically acceptable salt thereof used for improving muscle function or preventing, ameliorating or treating a muscle disease.

In relation to the second aspect, the present invention also provides the use of cyclo(his-pro) or a pharmaceutically or sitologically acceptable salt thereof for the manufacture of a medicament or health functional food for improving muscle function or preventing, ameliorating or treating a muscle disease.

In the present invention, the description of cyclo(his-pro) used as an effective ingredient is the same as that described in the first aspect, and therefore, the description thereof will be omitted.

In the present invention, the muscle disease is preferably a disease reported in the art as a muscle disease caused by muscle dysfunction, muscle atrophy, muscle loss or muscle degeneration, and it may be any one or more selected from the group consisting of atony, muscular atrophy, muscular dystrophy, myasthenia, cachexia, sarcopenia, myocardia and acardiotrophy, but is not limited thereto.

The muscle dysfunction, muscle atrophy, muscle loss or muscle degeneration may be caused by genetic factors, acquired factors, diseases causing muscle loss or weakening or aging, and according to one embodiment, it may be caused by aging. Alternatively, the muscle dysfunction, muscle atrophy, muscle loss or muscle degeneration may be a side effect of disease treatment, for example, a side effect of anticancer treatment. The muscle loss is characterized by a gradual loss of muscle mass, and weakening and degeneration of muscles, particularly skeletal muscle or voluntary muscle and cardiac muscle. Particularly, in the present invention, the "muscle disease" may be a disease in which muscle dysfunction, muscle atrophy, muscle loss or muscle degeneration occurs due to aging of the muscle itself, for example, skeletal muscle or cardiac muscle itself, regardless of the death of motor neurons in the central nervous system, or a disease in which muscle dysfunction, muscle atrophy, muscle loss or muscle degeneration occurs due to a genetic mutation.

As used herein, "old age" or "aging" is generally defined as a noticeable decline or loss of muscle mass beginning around age 50, and it may be sarcopenia (age-related muscle loss) becoming more pronounced around age 60.

In a specific embodiment of the present invention, changes in muscle weight and muscle fiber size were confirmed in an aged animal model treated with cyclo(his-pro).

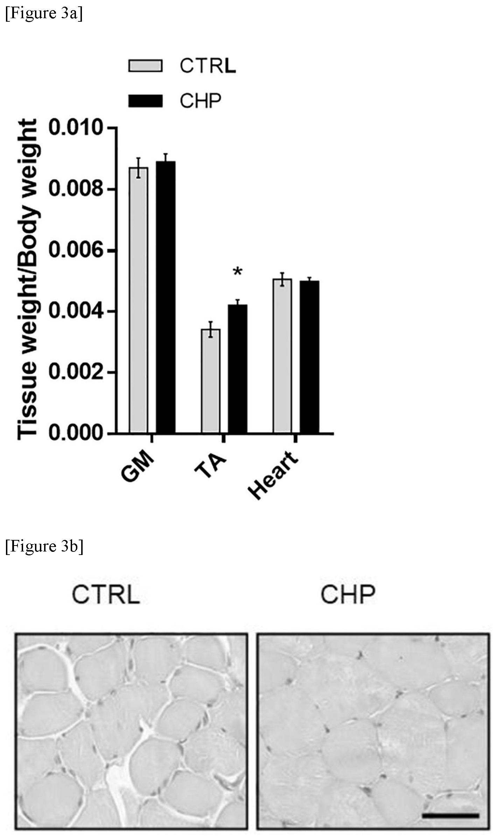

As a result, as shown in FIGS. 3A to 3D, it was confirmed that the cyclo(his-pro) administration group had an increase in the muscle weight of the tibialis anterior (TA) muscle and an increase in the average cross-sectional area of muscle fibers compared to the control group treated with the vehicle. Therefore, the cyclo(his-pro) according to the present invention may be effectively used for improving muscle function through an increase in muscle mass and muscle fiber size, as well as preventing, ameliorating or treating a muscle disease caused by decreased muscle function, muscle atrophy, muscle loss or muscle degeneration.

E3 ubiquitin ligase factors Atrogin-1/MAFbx (Muscle atrophy F-box) and MuRF-1 (Muscle RING-finger protein-1) are involved in protein degradation, and when their expression levels increase, protein degradation in muscles is promoted, resulting in a decrease in muscle mass.

PGC-1α is an important factor that regulates energy metabolism in muscles, regulating fatty acid oxidation and increasing energy levels by promoting mitochondrial biogenesis. In addition, PGC-1α is known to activate transcription factors that affect mitochondrial proliferation, energy homeostasis regulation and respiration, such as NRF1, TFAM, Sirt-1 and the like.

In addition, genes related to aging and muscle atrophy include Myostatin, Foxo-1, Dystrophin, Sirt-3 and Nrf-2, and known genes involved in mitochondrial biogenesis include Err-α, Cox-4, Drp-1, Tf1bm and Tf2bm.

Accordingly, in a specific embodiment of the present invention, the expression levels of the genes presented in Table 2 were determined in an aged animal model treated with cyclo(his-pro), thereby evaluating the effect of cyclo(his-pro) on improving muscle function and preventing, ameliorating or treating a muscle disease.

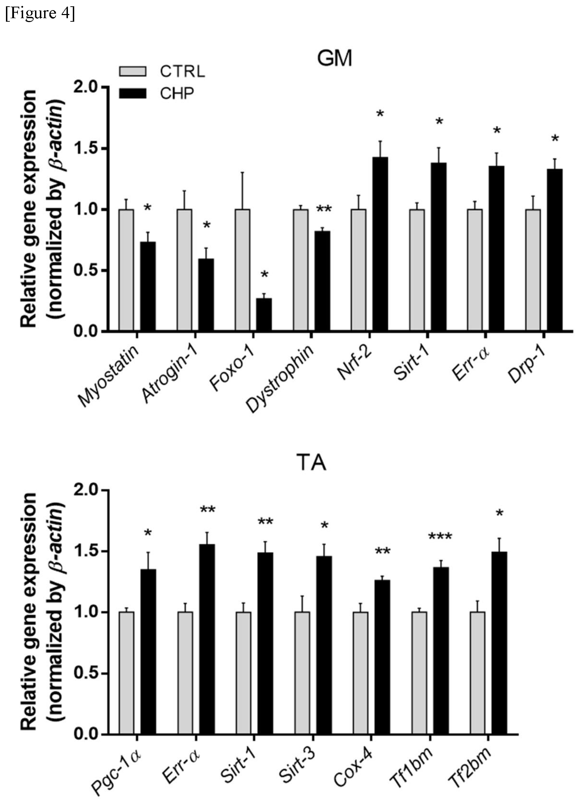

As a result, as shown in FIG. 4, the cyclo(his-pro) administration group showed a significant decrease in the gene expression levels of Myostatin, Atrogin-1, Foxo-1 and Dystrophin in skeletal muscle compared to the vehicle-treated control group, confirming that cyclo(his-pro) can be effectively used for the prevention, amelioration or treatment of muscle diseases caused by decreased muscle function, muscle atrophy, muscle loss or muscle degeneration through its muscle loss inhibition and muscle atrophy inhibition activities.

In addition, as shown in FIG. 4, it was confirmed that the gene expression levels of PGC-1α, Nrf-2, Sirt-1, Sirt-3, Drp-1, Err-α, Cox-4, Tf1bm and Tf2bm in skeletal muscle were significantly increased in the cyclo(his-pro) administration group compared to the vehicle-treated control group. Therefore, cyclo(his-pro) according to the present invention helps muscle energy production by increasing the expressions of mitochondrial-mediated energy metabolism regulators and suppresses muscle atrophy, thereby improving muscle function as well as treating muscle diseases caused by decreased muscle function, muscle atrophy, muscle loss or muscle degeneration.

Oxidation-related enzymes of the Krebs cycle (or TCA cycle) are utilized as biomarkers related to energy production and supply efficiency. Citrate synthase is an enzyme that catalyzes citrate synthesis in the first step of the TCA cycle, and if the TCA cycle does not run smoothly, blood lactate accumulates, thereby causing fatigue.

Accordingly, in a specific embodiment of the present invention, the activity change of citrate synthase was determined in an aged animal model treated with cyclo(his-pro), thereby evaluating the effects of cyclo(his-pro) on enhancing exercise performance and muscle function, and preventing, ameliorating or treating muscle diseases.

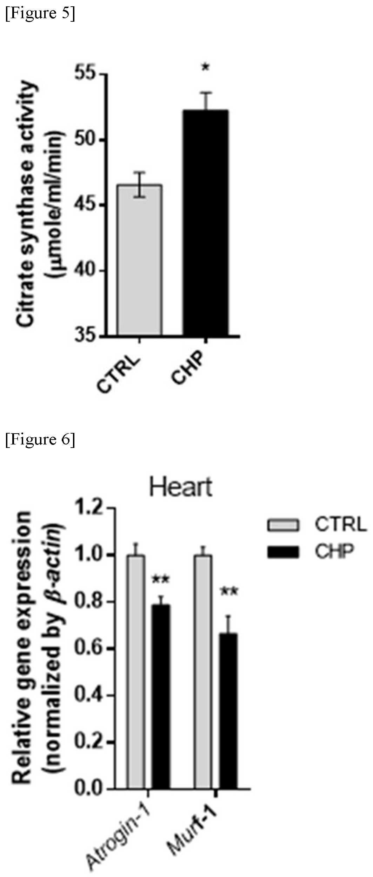

As a result, as shown in FIG. 5, the cyclo(his-pro) administration group was confirmed to be effective in enhancing exercise performance, improving muscle function, and preventing, ameliorating or treating a muscle disease through promoting recovery from muscle fatigue and increasing energy production and supply efficiency, by increasing the activity of citrate synthase in skeletal muscle, compared to the vehicle-treated control group.

In another specific embodiment of the present invention, in order to evaluate whether cyclo(his-pro) can inhibit muscle loss and muscle atrophy of not only skeletal muscle but also cardiac muscle, the gene expression levels of Atrogin-1 and Murf-1 were determined in cardiac muscle of an aged animal model treated with cyclo(his-pro).

As a result, as shown in FIG. 6, the cyclo(his-pro) administration group significantly reduced the gene expression levels of Atrogin-1 and Murf-1 in the myocardium compared to the vehicle-treated control group, thereby confirming that it can be effectively used for the prevention, amelioration or treatment of diseases caused by myocardial abnormalities through the inhibition of myocardial muscle loss and myocardial muscle atrophy inhibition activities.

In the present invention, the disease occurring due to an abnormality in the heart muscle may be, for example, cardiomyopathy or cardiac atrophy occurring primarily in the heart muscle itself, but is not limited thereto.

Acardiotrophy is caused by starvation, wasting disease (cancer, etc.) and aging, and the myocardial fibers become thin and thin, and the nuclei become condensed and irregular in size. Accordingly, the muscle fascicles also decrease in volume, the entire heart becomes smaller, the subepicardial adipose tissue is markedly reduced, and the coronary arteries become curved. A brown pigment called a wasting pigment (lipofuscine) appears at both ends of the nuclei of the myocardial fibers, and with the reduction of adipose tissue, the entire heart takes on a brownish tone.

The most common muscular dystrophy is X chromosome-linked Duchenne/Becker muscular dystrophy, but there are also several other types, including limb-girdling, fascioscapulohumeral, myotonic and Fukuyama, depending on the type of protein that makes up the muscle membrane.

Duchenne muscular dystrophy (DMD) is one of the most devastating and progressive inherited muscular dystrophies that appears in childhood. It is caused by mutations in the dystrophin gene and is a recessive X-linked genetic disorder that affects 1 in 3,500 male births. Notably, female carriers can also develop the disease. Dystrophin is a key member of the dystrophin-associated protein complex (DAPC), which is a basic link between the cytoskeleton and the extracellular matrix (ECM) of muscle fibers. Without functional dystrophin, DAPC is degraded, resulting in the loss of stable cytoskeleton-ECM connections, thereby weakening the sarcolemma and predisposing muscle fibers to contractile damage.

The first clinical signs appear in childhood, between the ages of 2 and 3, and children show motor developmental delays, with difficulty walking or jumping. Dystrophy first affects the proximal muscles and then spreads to the distal limb muscles, and children become wheelchair-bound by the age of 12. Dystrophy eventually reaches the respiratory muscles, requiring artificial ventilation. The heart muscle is also weakened, ultimately resulting in cardiomyopathy. In fact, respiratory and heart failure are the two main causes of death in DMD.

Despite all the therapeutic advances over the past several decades, there is still no approved cure for DMD. Nevertheless, improvements in treatment protocols and symptom management can delay the progression of DMD and improve the quality of life of patients. With proper treatment, the life expectancy of DMD patients today is approximately 28 years. The current standard of care is corticosteroid therapy, which reduces chronic inflammation and delays muscle damage caused by inflammation. In particular, long-term use of corticosteroids has several side effects, including weight gain, growth retardation and osteoporosis.

The most widely used animal model of DMD is the Dmdmdx (mdx) mouse. This model, which is generated on a C57BL/10ScSn background, carries a nonsense mutation in exon 23 of the dystrophin gene, creating an early stop codon and thus a truncated form of the protein.

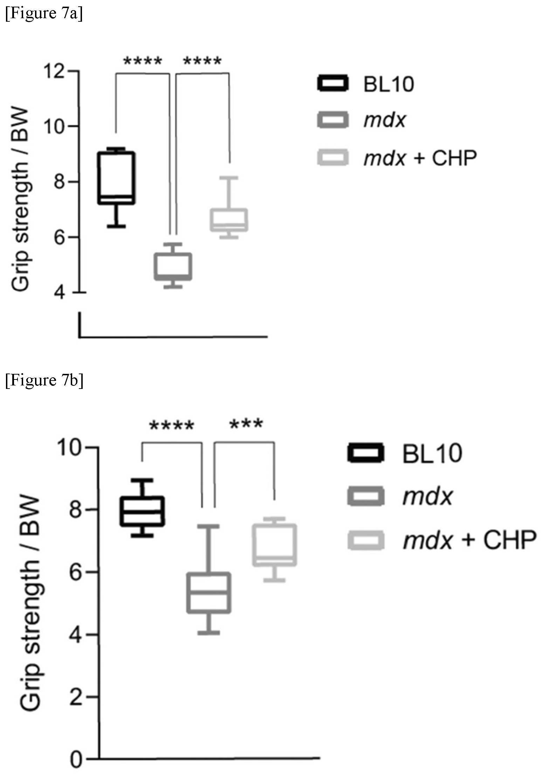

In a specific embodiment of the present invention, the effect of cyclo(his-pro) on enhancing exercise performance was evaluated by performing grip strength measurement and limb hanging test according to the cyclo(his-pro) prevention and treatment protocols in an mdx mouse model. As a result, as shown in FIGS. 7A, 7B and 8, it was confirmed that the cyclo(his-pro) administration group significantly increased grip strength and hanging time compared to the mdx control group treated with water, thereby showing the effects of increasing limb muscle strength and exercise endurance.

In another specific embodiment of the present invention, the contractile and force production effects were evaluated in extensor digitorum longus (EDL) and soleus muscles isolated from an mdx mouse model to which a cyclo(his-pro) prevention protocol was applied. As a result, as shown in FIGS. 9A to 9F, the muscles of the mdx mouse showed overall lower force production and a downward shift in the force-frequency relationship compared to the BL10 mouse. The performance of the EDL and soleus muscles was improved by CHP, and the maximal contractile force was partially restored. This suggests that the known cyclo(his-pro) treatment maintains force production.

In still another specific embodiment of the present invention, in order to evaluate the effect of cyclo(his-pro) on Ca²⁺ dysregulation, the effect of restoring Ca²⁺ release/uptake from the sarcoplasmic reticulum (SR) of flexor digitorum brevis (FDB) muscle isolated from the mdx mouse model to which the cyclo(his-pro) prevention protocol was applied was evaluated. As a result, as shown in FIG. 10A, the response to caffeine-induced Ca²⁺ release in the SR was recovered by cyclo(his-pro), and as shown in FIG. 10B, although Ca²⁺ uptake through store-operated calcium channels (SOC) after SR depletion was generally upregulated in mdx as a compensatory mechanism for Ca2⁺ depletion, CHP normalized Ca²⁺ uptake, thereby maintaining appropriate Ca²⁺ handling. Additionally, in order to directly quantify SOCE, the stimulation of SR Ca²⁺ release was performed by using thapsigargin instead of caffeine, and as a result, a similar trend of cyclo(his-pro)-induced normalization was confirmed, as shown in FIGS. 10C and 10D.

In addition, cyclo(his-pro) or a salt thereof according to the present invention may be administered to a subject for the purpose of improving muscle function or preventing, ameliorating or treating a muscle disease. Accordingly, provided is a method for improving muscle function or preventing, ameliorating or treating a muscle disease, including administering cyclo(his-pro) or a salt thereof to a subject in need thereof.

A third aspect of the present invention relates to a composition for strengthening muscle strength, comprising cyclo(his-pro) or a salt thereof, consisting essentially of the cyclo(his-pro) or a salt thereof, or consisting of the cyclo(his-pro) or a salt thereof.

Specifically, the composition may be a pharmaceutical composition for strengthening muscle strength, comprising cyclo(his-pro) or a pharmaceutically acceptable salt thereof, consisting essentially of the cyclo(his-pro) or a pharmaceutically acceptable salt thereof, or consisting of the cyclo(his-pro) or a pharmaceutically acceptable salt thereof; a health functional food composition for strengthening muscle strength, comprising cyclo(his-pro) or a sitologically acceptable salt thereof, consisting essentially of the cyclo(his-pro) or a sitologically acceptable salt thereof, or consisting of the cyclo(his-pro) or a sitologically salt thereof; or a feed additive for strengthening muscle strength, comprising cyclo(his-pro) or a salt thereof, consisting essentially of the cyclo(his-pro) or a salt thereof, or consisting of the cyclo(his-pro) or a salt thereof.

In relation to the third aspect, the present invention provides a method for strengthening muscle strength, including administering an effective amount of cyclo(his-pro) or a pharmaceutically or sitologically acceptable salt thereof to a subject in need thereof.

In relation to the third aspect, the present invention also provides the use of cyclo(his-pro) or a pharmaceutically or sitologically acceptable salt thereof used for strengthening muscle strength.

In relation to the third aspect, the present invention also provides the use of cyclo(his-pro) or a pharmaceutically or sitologically acceptable salt thereof for the manufacture of a medicament or health functional food for strengthening muscle strength.

In the present invention, the description of cyclo(his-pro) used as an effective ingredient is the same as that described in the first aspect, and therefore, the description thereof will be omitted.

The term "strengthening muscle strength" of the present invention refers to the effects of enhancing physical performance, enhancing maximum endurance, increasing muscle mass, enhancing muscle recovery, reducing muscle fatigue, improving energy balance or a combination thereof.

Cyclo(his-pro) or a salt thereof according to the present invention not only exhibits the effects of increasing muscle strength, increasing movement coordination, increasing resistance to muscle fatigue, and increasing a sense of balance as described in the first and second aspects, but also exhibits the effects of increasing muscle mass, increasing muscle fiber size, inhibiting muscle atrophy, inhibiting muscle loss and increasing muscle energy production, and thus can be utilized in various ways as a pharmaceutical product, a health functional food product and a feed additive for the purpose of strengthening muscle strength.

In addition, cyclo(his-pro) or a salt thereof according to the present invention may be administered to a subject for the purpose of strengthening muscle strength. Accordingly, provided is a method for strengthening muscle strength, including administering cyclo(his-pro) or a salt thereof to a subject in need thereof.

As used herein, the term "prevention" means any act of inhibiting or delaying the onset of a muscle disease by administering a composition according to the present invention.

As used herein, the term "amelioration" means any action that at least reduces a parameter related to the condition being treated, for example, the degree of symptoms.

As used herein, the term "treatment" means any action by which symptoms of a muscle disease are improved, symptoms are prevented from worsening, or symptoms are beneficially changed by the administration of a composition according to the present invention.

As used herein, the term "increase," means, for example, an increase in the expression of a specific biomarker gene or protein, which is about 5% or more, about 10% or more, about 15% or more, about 20% or more, about 25% or more, about 30% or more, about 35% or more, about 40% or more, about 45% or more, about 50% or more higher, compared to a control group that is not administered the active ingredient or a composition comprising, consisting essentially of, or consisting of the active ingredient of the present invention under the same conditions.

As used herein, the term "decrease or reduction" or "suppression", for example, a decrease or suppression of the expression of a specific biomarker gene or protein, means a decrease of about 5% or more, about 10% or more, about 15% or more, about 20% or more, about 25% or more, about 30% or more, about 35% or more, about 40% or more, about 45% or more, about 50% or more, about 55% or more, about 60% or more, about 65% or more, about 70% or more, about 75% or more, compared to a control group that is not administered the active ingredient or composition comprising, consisting essentially of, or consisting of the active ingredient of the present invention, under the same conditions.

As used herein, "enhancement or improvement", for example, enhancement in exercise performance or improvement in muscle function, means an increase of about 5% or more, about 10% or more, about 15% or more, about 20% or more, compared to a control group that is not administered the active ingredient or the composition comprising, consisting essentially of, or consisting of the active ingredient of the present invention under the same conditions.

In the present invention, the term "pharmaceutically acceptable" means physiologically acceptable and does not typically cause an allergic reaction or similar reaction when administered to a human, and the salt is preferably an acid addition salt formed by a pharmaceutically acceptable free acid.

The pharmaceutically acceptable salt may be an acid addition salt formed by using an organic acid or an inorganic acid, and the organic acid includes, for example, formic acid, acetic acid, propionic acid, lactic acid, butyric acid, isobutyric acid, trifluoroacetic acid, malic acid, maleic acid, malonic acid, fumaric acid, succinic acid, succinic acid monoamide, glutamic acid, tartaric acid, oxalic acid, citric acid, glycolic acid, glucuronic acid, ascorbic acid, benzoic acid, phthalic acid, salicylic acid, anthranilic acid, dichloroacetic acid, aminooxy acetic acid, benzenesulfonic acid, p-toluenesulfonic acid or methanesulfonic acid. The inorganic acid includes, for example, hydrochloric acid, hydrobromic acid, sulfuric acid, phosphoric acid, nitric acid, carbonic acid or boric acid. The acid addition salt may preferably be in the form of a hydrochloride or an acetate salt, and more preferably in the form of a hydrochloride salt.

In addition, additional possible salt forms include gabapentin salt, gabapentin salt, pregabalin salt, nicotinate salt, adipate salt, hemimalonate salt, cysteine salt, acetylcysteine salt, methionine salt, arginine salt, lysine salt, ornithine salt or aspartate salt, etc.

In addition, the pharmaceutical composition of the present invention may further include a pharmaceutically acceptable carrier. The pharmaceutically acceptable carrier may further include, for example, a carrier for oral administration or a carrier for parenteral administration. The carrier for oral administration may include lactose, starch, cellulose derivatives, magnesium stearate, stearic acid and the like. The carrier for parenteral administration may include water, suitable oils, saline, aqueous glucose and glycols and the like. In addition, the composition may further include a stabilizer and a preservative. Suitable stabilizers include antioxidants such as sodium bisulfite, sodium sulfite or ascorbic acid. Suitable preservatives include benzalkonium chloride, methyl- or propyl-paraben and chlorobutanol. Other pharmaceutically acceptable carriers may be referred to those described in the following literature (Remington's Pharmaceutical Sciences, 19th ed., Mack Publishing Company, Easton, PA, 1995).

The pharmaceutical composition of the present invention may be administered to mammals, including humans, by any method. For example, it may be administered orally or parenterally, and parenteral administration methods include, but are not limited to, intravenous, intramuscular, intraarterial, intramedullary, intrathecal, intracardiac, transdermal, subcutaneous, intraperitoneal, intranasal, enteral, topical, sublingual or rectal administration.

The pharmaceutical composition of the present invention may be formulated as a preparation for oral administration or parenteral administration according to the administration route as described above. When formulated, it may be prepared by using one or more buffers (e.g., saline or PBS), carbohydrates (e.g., glucose, mannose, sucrose, or dextran, etc.), antioxidants, bacteriostats, chelating agents (e.g., EDTA or glutathione), fillers, bulking agents, binders, adjuvants (e.g., aluminum hydroxide), suspending agents, thickening agents, wetting agents, disintegrating agents or surfactants, diluents or excipients.

Solid preparations for oral administration include tablets, pills, powders, granules, liquids, gels, syrups, slurries, suspensions or capsules, and these solid preparations may be prepared by mixing the pharmaceutical composition of the present invention with at least one excipient, for example, starch (including corn starch, wheat starch, rice starch, potato starch, etc.), calcium carbonate, sucrose, lactose, dextrose, sorbitol, mannitol, xylitol, erythritol maltitol, cellulose, methyl cellulose, sodium carboxymethyl cellulose, and hydroxypropyl methyl-cellulose or gelatin. For example, a tablet or a sugar-coated tablet may be obtained by mixing an active ingredient with a solid excipient, grinding the mixture, adding a suitable auxiliary agent, and then processing into a granule mixture.

In addition to simple excipients, lubricants such as magnesium stearate and talc are also used. Liquid preparations for oral administration include suspensions, solutions, emulsions or syrups, and may include various excipients such as wetting agents, sweeteners, flavoring agents or preservatives in addition to the commonly used simple diluents such as water or liquid paraffin.

In addition, cross-linked polyvinylpyrrolidone, agar, alginic acid or sodium alginate may be added as a disintegrating agent depending on the case, and anticoagulants, lubricants, wetting agents, fragrances, emulsifiers and preservatives may be additionally included.

When administered parenterally, the pharmaceutical composition of the present invention may be formulated in the form of injections, transdermal administration agents and nasal inhalers together with a suitable parenteral carrier according to methods known in the art. In the case of the injection, it must be sterilized and protected from contamination by microorganisms such as bacteria and fungi. Examples of suitable carriers for injection include, but are not limited to, solvents or dispersion media including water, ethanol, polyols (e.g., glycerol, propylene glycol, and liquid polyethylene glycol), mixtures thereof, and/or vegetable oils. More preferably, suitable carriers include Hanks' solution, Ringer's solution, PBS (phosphate buffered saline) containing triethanolamine or isotonic solutions such as sterile water for injection, 10% ethanol, 40% propylene glycol and 5% dextrose. In order to protect the injection from microbial contamination, various antibacterial and antifungal agents such as paraben, chlorobutanol, phenol, sorbic acid, thimerosal and the like may be additionally included. In addition, the injection may in most cases additionally include an isotonic agent such as sugar or sodium chloride.

In the case of percutaneous administration, the forms include ointments, creams, lotions, gels, external solutions, pastes, liniments, aerosols and the like. As used above, 'percutaneous administration' means topically administering a pharmaceutical composition to the skin such that an effective amount of active ingredients contained in the pharmaceutical composition is delivered into the skin.

For inhalation administration, the compound used according to the invention may conveniently be delivered in the form of an aerosol spray from a pressurized pack or nebulizer, using a suitable propellant, for example, dichlorofluoromethane, trichlorofluoromethane, dichlorotetrafluoroethane, carbon dioxide or other suitable gas. In the case of a pressurized aerosol, the dosage unit may be determined by providing a valve that delivers a metered amount. For example, gelatin capsules and cartridges for use in an inhaler or insufflator may be formulated so as to contain a powder mixture of the compound and a suitable powder base such as lactose or starch. Formulations for parenteral administration are described in the well-known prescription literature of pharmaceutical chemistry (Remington's Pharmaceutical Science, 15th Edition, 1975. Mack Publishing Company, Easton, Pennsylvania 18042, Chapter 87: Blaug, Seymour).

The pharmaceutical composition of the present invention may provide a desirable effect of enhancing exercise performance, improving muscle function and/or preventing, ameliorating or treating a muscle disease when it includes an effective amount of cyclo(his-pro) or a pharmaceutically acceptable salt thereof. As used herein, the term "effective amount" refers to an amount that shows a greater response than a negative control, and preferably refers to an amount that is sufficient to enhance exercise performance, improve muscle function, and/or prevent, ameliorate or treat a muscle disease. The pharmaceutical composition of the present invention may include 0.01 to 99.9% of cyclo(his-pro) or a pharmaceutically acceptable salt thereof based on the total amount of the composition, and the remainder may be included of a pharmaceutically acceptable carrier. The effective amount of cyclo(his-pro) or a pharmaceutically acceptable salt thereof included in the pharmaceutical composition of the present invention will vary depending on the form in which the composition is commercialized and the like.

The total effective amount of the pharmaceutical composition of the present invention may be administered to a patient as a single dose, or may be administered by a fractionated treatment protocol in which multiple doses are administered over a long period of time. The pharmaceutical composition of the present invention may vary the content of the effective ingredient depending on the condition of the patient. For example, based on cyclo(his-pro) or a pharmaceutically acceptable salt thereof, it may be administered in one to several divided doses, preferably in an amount of 0.001 to 100 mg, and more preferably 0.01 to 10 mg per kg of body weight per day. However, since the dosage of the cyclo(his-pro) or a pharmaceutically acceptable salt thereof is determined as an effective dosage for a patient by taking into consideration various factors such as the route of administration and the number of treatments of the pharmaceutical composition as well as the patient's age, weight, health condition, sex, severity of disease, diet and excretion rate, taking these points into consideration, a person having ordinary skill in the art will be able to determine an appropriate effective dosage of the cyclo(his-pro) or a pharmaceutically acceptable salt thereof for a specific use for enhancing exercise performance, improving muscle function and/or preventing, ameliorating or treating a muscle disease. The pharmaceutical composition according to the present invention is not particularly limited in its formulation, administration route and administration method as long as it exhibits the effects of the present invention.

In the present invention, the term "sitologically acceptable" means physiologically acceptable and does not typically cause an allergic reaction or similar reaction when ingested by humans, and the salt is preferably an acid addition salt formed by a sitologically acceptable free acid.

In the present invention, preferred examples of "a sitologically acceptable salt" may include the types of "pharmaceutically acceptable salts" described above.

In the present invention, the term "health functional food" includes both the meanings of "functional food" and "health food".

In the present invention, the term "functional food" is the same as food for special health use (FoSHU), and means a food with high medical and healthcare effects that is processed to efficiently exhibit a bioregulatory function in addition to providing nutrition.

In the present invention, the term "health food" means a food that has a more active health maintenance or promotion effect than general food, and health supplement food means a food for the purpose of health supplementation. In some cases, the terms functional food, health food and health supplement food are used interchangeably. The food may be manufactured in various forms such as tablets, capsules, powders, granules, liquids and pills.

As a specific example of such functional foods, processed foods may be manufactured by using the composition to transform and preserve the characteristics of agricultural, livestock or marine products while improving their storage properties.

The health functional food composition of the present invention may also be manufactured in the form of nutritional supplements or dietary supplements, food additives and the like, and is intended for human consumption.

The food composition of the above type may be manufactured in various forms according to conventional methods known in the art. General foods include, but are not limited to, beverages (including alcoholic beverages), fruits and processed foods thereof (e.g., canned fruits, bottled fruits, jams, marmalades, etc.), fish, meats and processed foods thereof (e.g., ham, sausages, corned beef, etc.), breads and noodles (e.g., udon, buckwheat noodles, ramen, spaghetti, macaroni, etc.), fruit juices, various drinks, cookies, taffy, dairy products (e.g., butter, cheese, etc.), edible plant fats, margarine, vegetable proteins, retort foods, frozen foods, various seasonings (e.g., soybean paste, soy sauce, sauces, etc.) and the like, which can be manufactured by adding cyclo(his-pro) or a sitologically acceptable salt thereof.

In addition, nutritional supplements may be manufactured by adding cyclo(his-pro) or a sitologically acceptable salt thereof to capsules, tablets, pills and the like, but are not limited thereto.

In addition, as a health functional food, although not limited thereto, for example, the cyclo(his-pro) or a sitologically acceptable salt thereof may be manufactured in the form of tea, juice and drink and consumed (health beverage) by liquefying, granulating, encapsulating and powdering. Additionally, in order to use the cyclo(his-pro) or a sitologically acceptable salt thereof in the form of a food additive, it may be manufactured in the form of a powder or concentrate and used. In addition, the cyclo(his-pro) or a sitologically acceptable salt thereof may be manufactured in the form of a composition by mixing with a known active ingredient that is known to be effective in enhancing exercise performance, improving muscle function and/or preventing, ameliorating or treating a muscle disease.

When the food composition of the present invention is used as a health beverage composition, the health beverage composition may contain various flavoring agents or natural carbohydrates as additional ingredients, like a typical beverage. The above-described natural carbohydrates may be monosaccharides such as glucose and fructose; disaccharides such as maltose and sucrose; polysaccharides such as dextrin and cyclodextrin; and sugar alcohols such as xylitol, sorbitol and erythritol. The sweetener may be a natural sweetener such as thaumatin and stevia extract; a synthetic sweetener such as saccharin and aspartame and the like. The proportion of the natural carbohydrate is generally about 0.01 to 0.04 g, and preferably about 0.02 to 0.03 g, per 100 mL of the composition of the present invention.

Cyclo(his-pro) or a sitologically acceptable salt thereof may be contained as an effective ingredient in a food composition for enhancing exercise performance, improving muscle function, and/or preventing or ameliorating a muscle disease, and the amount thereof is an amount effective to obtain the above effects, and is preferably, for example, 0.01 to 100 wt% based on the total weight of the entire composition, but is not particularly limited thereto.

In addition to the above, the health functional food of the present invention may contain various nutrients, vitamins, electrolytes, flavoring agents, coloring agents, pectic acid, salts of pectic acid, alginic acid, salts of alginic acid, organic acids, protective colloid thickeners, pH regulators, stabilizers, preservatives, glycerin, alcohols or carbonating agents. In addition, the health functional food of the present invention may contain fruit pulp for the production of natural fruit juice, fruit juice drinks or vegetable drinks. These ingredients may be used independently or in mixtures. The ratio of these additives is not particularly important, but is generally selected in the range of 0.01 to 0.1 parts by weight per 100 parts by weight of the composition of the present invention.

In the present invention, the term "feed" means a substance that supplies organic or inorganic nutrients that are necessary for sustaining the life of an animal. The feed includes nutrients such as energy, protein, lipid, vitamins and minerals required by animals such as livestock, and may be plant-based feed such as grains, roots, fruits, food processing by-products, algae, fibers, fats, starches, meal, and grain by-products, or animal-based feed such as proteins, inorganic substances, fats, minerals, fats and single-cell proteins, but is not limited thereto.

In the present invention, "a feed additive" means a substance added to feed to improve animal productivity or health, and is not particularly limited thereto, but may additionally include amino acids, vitamins, enzymes, flavoring agents, silicates, buffers, extractants, oligosaccharides and the like for growth promotion, disease prevention, etc.

The content of cyclo(his-pro) or a salt thereof included in the feed additive of the present invention is not particularly limited, but may be, for example, 0.001 to 1 % (w/w), 0.005 to 0.9 % (w/w), or 0.01 to 0.5 % (w/w).

Preferred examples of "a salt of cyclo(his-pro)" included in the feed additive of the present invention may include the types of the "pharmaceutically acceptable salt" and the "sitologically acceptable salt" described above.

As used herein, the term "subject" refers to a normal subject in need of enhancement in exercise performance, improvement in muscle function or enhancement of muscle strength, as well as a subject that has already developed or may develop a muscle disease, and the subject refers to all mammals including humans, dogs, cows, horses, rabbits, mice, rats, chickens or humans, but the mammals of the present invention are not limited by the above examples. This term does not indicate a specific age or gender. Therefore, it is intended to include adult/adult and newborn subjects, as well as fetuses, whether female/female or male/male. A patient refers to a subject suffering from a disease or disorder. The term patient includes human and veterinary subjects.

In one embodiment, the subject may be a human being aged at least about 50 years, at least about 55 years, at least about 60 years, or at least about 65 years. In another embodiment, the subject may exclude a human being aged at least about 50 years, at least about 55 years, at least about 60 years, or at least about 65 years.

In another embodiment, the subject may be a human patient with type 2 diabetes having excessive loss of skeletal muscle. In still another embodiment, the subject may be a human patient with excessive loss of skeletal muscle but not having diabetes. In still another embodiment, the subject may exclude a human patient with diabetes.

In still another embodiment, the subject may be a human patient suffering from muscle dysfunction, muscle atrophy, muscle loss or muscle degeneration due to a genetic mutation.

In still another embodiment, the subject may be a patient who has received, is receiving or will receive anticancer treatment, particularly chemotherapy.

In another embodiment, the subject is a subject in need of improved exercise performance, which may be in a state of poor or insufficient exercise performance relative to a particular training requirement. The subject in need of improved exercise performance may be experiencing symptoms of fatigue and/or muscle fatigue. The subject in need of improved exercise performance may be a subject engaged in physical exertion, particularly in an athletic event or competition.

In another embodiment, the subject may be a subject who desires to improve or enhance athletic performance. The subject who desires to enhance athletic performance may not be a subject who lacks or is not insufficient in athletic performance, but may be a subject who desires to increase or enhance athletic performance compared to his or her normal state. The subject who needs improved athletic performance may be a subject who is particularly interested in performing physical exertion in an athletic game or competition.

The optimal dosage and dosing interval for individual administration of cyclo(his-pro) or a salt thereof will be determined by the nature and extent of the disease being treated, the dosage form, route and site of administration, and the age and health of the particular patient being treated, and it will be appreciated by those skilled in the art that the physician will ultimately determine the appropriate dosage to be used. Such administrations may be repeated as often as appropriate. If side effects occur, the dosage and frequency may be modified or reduced in accordance with routine clinical practice.

The route of administration of cyclo(his-pro) or a salt thereof may be administered via any common route as long as it can reach the target tissue. The cyclo(his-pro) or a salt thereof according to the present invention may be administered intraperitoneally, intravenously, subcutaneously, intradermally or orally, depending on the purpose, but is not limited thereto. In addition, the cyclo(his-pro) or a salt thereof may be administered by any device that is capable of transporting to the target cell.

### MODES OF THE INVENTION

Hereinafter, the present invention will be described in more detail through examples. However, the present invention can be modified in various ways and can have various forms, and the specific examples and descriptions described below are only intended to help understand the present invention, and are not intended to limit the present invention to specific disclosed forms. It should be understood that the scope of the present invention includes all modifications, equivalents or substitutes included in the spirit and technical scope of the present invention.

### [Example 1]

### Evaluation of muscle function improvement according to CHP treatment

### 1-1. Breeding of laboratory animals and CHP administration

For experiments on older animals, 18-month-old C57BL6/J male mice were purchased from the Korea Basic Science Institute (KSBI). Mouse feed from Purina was purchased, and cyclo(his-pro) (CHP) was purchased from Bachem.

Mice were housed in a constant temperature and humidity chamber maintained at 23±3°C, 50% humidity and a 12-h light/dark cycle with free access to food and water. The mice were weighed and randomly distributed to have equal average weights and divided into two groups as shown in Table 1. The CHP administration group was administered 35 mg/kg of CHP orally once/day for 4 months. The control group was administered the same amount (200 µL) of distilled water.

**[Table 1]**

| **Group** | Control | **CHP administration group** |
|---|---|---|
| **Administration (oral)** | Vehicle (distilled water) | CHP (35 mg/kg) |
| **Number of mice** | 7 | 7 |

### 1-2. Survival rate

As confirmed in FIG. 1, the control group showed a survival rate of 71.4% with two mice dying of natural aging during the experimental period, but the CHP administration group showed normal vital signs and a survival rate of 100%.

### 1-3. Grip strength test

In order to measure the change in muscle strength due to CHP, a grip strength test was performed on animals in the control and CHP administration groups. The maximum grip strength of the forepaws and hind paws that appeared when the tail of the mouse was held and the mouse was made to hold the grid of the grip strength test device and the tail was pulled backward was measured by using a grip strength meter (Bioseb). The grip strength was measured repeatedly three times, and the average value was used after being corrected for body weight. As shown in FIG. 2a, the maximum muscle strength of the CHP administration group significantly increased by about 23.6% compared to the control group.

### 1-4. Four limb-wire hanging test

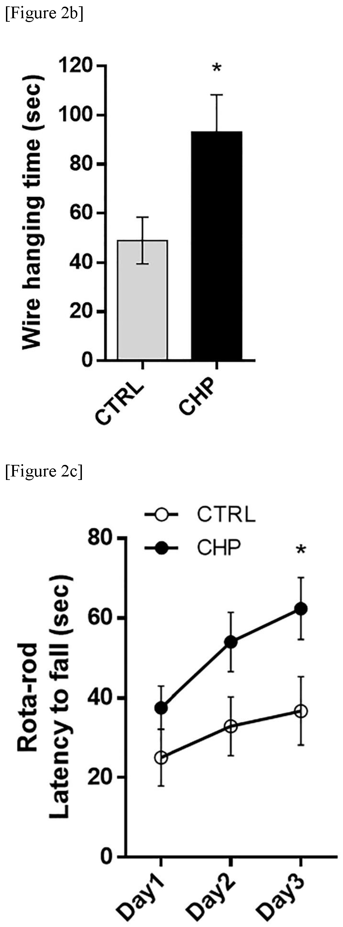

The four limb-wire hanging test is a muscle function measurement method that comprehensively measures muscle strength, coordination and tolerance to fatigue in rodents, and is a method to measure the continuous muscle function of mice against body weight (Lee et al., The Effects of 8-week Acetic Acid Feeding on Endurance Performance and Fat Metabolism in Skeletal Muscle of Mice, Exerc Sci, 2021). The mouse was placed on a 10x10 cm wire grid and carefully turned over, and the time until it fell to the floor was measured. The height to the floor was set to 40 cm, and a cushion was placed on the floor where the mouse fell to prevent shock caused by falling. The longest hanging time among the two measurements was recorded. As a result of the experiment, as shown in FIG. 2b, the hanging time of the CHP administration group significantly increased by about 1.9 times compared to the control group.

### 1-5. Evaluation of sensorimotor function (Rota-rod test)

The evaluation of sensorimotor function is a method to measure motor coordination and balance in animals (Robert M J Deacon, Measuring motor coordination in mice, J Vis Exp, 2013). The mouse was placed on a rotating cylinder of a Rota-rod device (Harvard Apparatus) that rotated at 4 rpm, and the speed was steadily increased to 40 rpm for 5 minutes, and the time until they lost their balance and fell to the floor was measured. The experiment was conducted 3 times/day for a total of 3 days, and the average value of the 3 measurements was used. As shown in FIG. 2c, the CHP administration group showed improved sensorimotor function compared to the control group, and statistical significance was confirmed on day 3.

From the above results, it was found that CHP intake significantly improved muscle strength, motor coordination, resistance to muscle fatigue and sense of balance.

### [Example 2]

### Changes in muscle weight and muscle fiber size according to CHP treatment

### 2-1. Measurement of muscle weight

When the mice of Example 1 reached 24 months of age, the mice were euthanized, and the gastrocnemius (GM), tibialis anterior (TA) and cardiac muscle tissues were isolated. The weights of each tissue were measured and compared, and as shown in FIG. 3a, it was confirmed that the weight of the TA muscle significantly increased due to CHP administration.

### 2-2. Measurement of muscle fiber size improvement

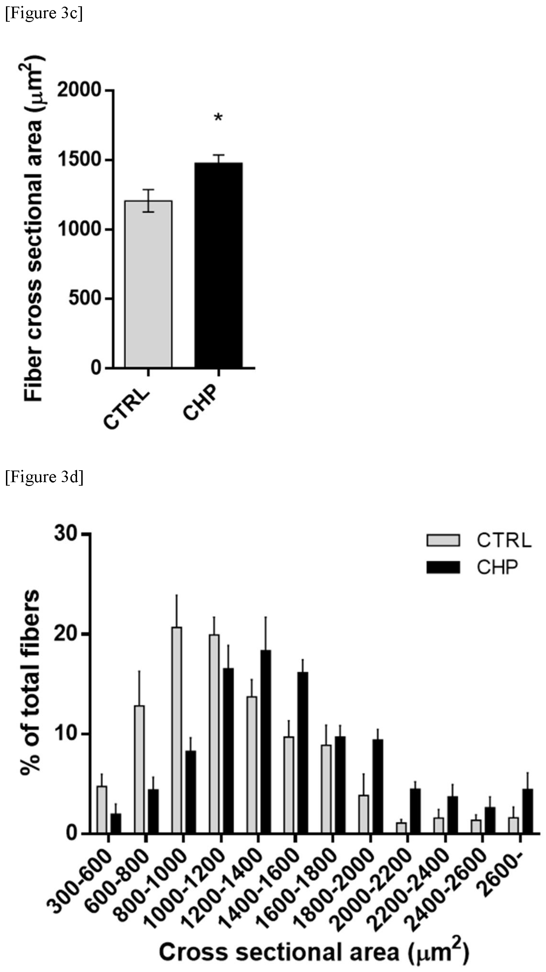

Formalin-fixed GM tissues were sliced into paraffin sections, and the muscle fibers were stained by using H&E staining. As a result of measuring the cross-sectional area of each muscle fiber, as shown in FIGS. 3b, 3c and 3d, it was confirmed that in the CHP administration group, the distribution of muscle fibers over a wide area increased, and the average cross-sectional area of muscle fibers significantly increased by about 22% compared to the control group.

From the above results, it was found that CHP had a positive effect on changes in muscle fiber size as well as quantitative changes in muscles.

### [Example 3]

### Measurement of changes in muscle gene expression according to CHP treatment

Muscle tissue was extracted for RNA by using NucleoZOL (MACHEREY-NAGEL) according to the manufacturer's total RNA isolation protocol, and 1 µg of RNA was subjected to reverse transcription polymerase chain reaction (RT-PCR) to synthesize cDNA by using ReverTra Ace qPCR RT Master Mix (Toyobo). The synthesized cDNA was analyzed by real-time PCR using primer sets of *myostatin, atrogin-1, foxo-1, dystrophin, sirt-1, sirt-3 and nrf-2* genes that are known to be related to aging and muscle atrophy and SYBR Green Realtime PCR Master mix (Toyobo) to quantify the expression level. The expression value of each gene was corrected by dividing it by the expression value of the housekeeping gene *β-actin*. Each primer set was synthesized by request from Bioneer, and the base sequence information is shown in Table 2.

**[Table 2]**

| Gene | Forward (5'-3') | SEQ ID NO: | Reverse (5'-3') | SEQ ID NO: |
|---|---|---|---|---|
| *Myostatin* | ACCCGTCAAGACTCC TACAA | 1 | CCTGGGCTCATGTCAAG TTT | 2 |
| *Atrogin-1* | TCAAAGGCCTCACG ATCACC | 3 | TCAAACGCTTGCGAATC TGC | 4 |
| *Foxo-1* | CCTTTCCTCCTCCCT CTG | 5 | TGCCTCTACTGAATGATT ACA | 6 |
| *Dystrophin* | AAGAGGAAGAAATG | 7 | CCATGCGGGAATCAGGA | 8 |
| | CCCCCG | | GTT | |
| *Drp-1* | AGGAGAAGAGGAAG CAAGCG | 9 | TAGGCTTTCCAGCACTG AGC | 10 |
| *Err-α* | CAGGAGGCAGACAC TGAT | 11 | CGGATTAAGCAGCAGCA A | 12 |
| *Sirt-1* | GTTGACCGATGGACT CCTCAC | 13 | GAGCTGGCGTGTGACGT TC | 14 |
| *Sirt-3* | ATGTCACTCACTACT TCCTG | 15 | ATCCCAGATGCTCTCTCA | 16 |
| *Nrf-2* | CAGCATAGAGCAGG ACATGGAG | 17 | GAACAGCGGTAGTATCA GCCAG | 18 |
| *Pgc-1α* | AAGGACTCTGAGAA CACTTG | 19 | CAACTGACCCAAACACT TTAC | 20 |
| *Cox-4* | TACTTCGGTGTGCCT TCGA | 21 | TGACATGGGCCACATCA G | 22 |
| *Tfb1m* | GGCTGAGAGACTTGT AGCCACT | 23 | AGGTGCACCACTCCTAC ATCAA | 24 |
| *Tfb2m* | TTTGGCAAGTGGCCT GTGAC | 25 | ACTGATTCCCCGTGCTTT GACT | 26 |
| *Murf-1* | GCTACCTTCCTCTCA AGTGC | 27 | CTCAAGGCCTCTGCTAT GTG | 28 |
| *β-actin* | GGGAAGGTGACAGC ATTG | 29 | ATGAAGTATTAAGGCGG AAGATT | 30 |

As a result of the experiment, as shown in FIG. 4, it was confirmed that the expression of genes related to aging and muscle atrophy was maintained to be lower in the CHP administration group compared to the control group. In particular, *sirt-1*, which is a representative anti-aging gene, was confirmed to increase equally in GM and TA muscles. Sirt-1 acts as a major regulator of energy and metabolic homeostasis and is known to be a major indicator reflecting the rate of cellular aging as it decreases with aging (Lagouge et al., Resveratrol improves mitochondrial function and protects against metabolic disease by activating SIRT1 and PGC-1alpha, Cell, 2006). Through this, it was found that CHP can suppress muscle aging and muscle atrophy. Moreover, as the expression of the nrf-2 gene, which is at the core position of the antioxidant action, was high in the CHP administration group, it was found that CHP also exhibited an antioxidant effect on muscles that increases resistance to oxidative stress.

Meanwhile, Sirt-1 is known to regulate pgc-1α, which is important for maintaining mitochondrial biogenesis and function (Handschin et al., Peroxisome Proliferator-Activated Receptor γ Coactivator 1 Coactivators, Energy Homeostasis, and Metabolism, Endocrine Reviews, 2006). In addition, the expressions of *Err-α, Cox-4, Drp-1, Tf1bm and Tf2bm,* which are genes related to mitochondrial biogenesis and function, were examined in GM and TA muscles. Likewise, the expression value of each gene was corrected by dividing it by the expression value of *β-actin,* which is a housekeeping gene. Each primer set was synthesized and used by Bioneer, and the base sequence information is shown in Table 2.

As a result of the experiment, as shown in FIG. 4, it was confirmed that the expressions of related genes significantly increased by CHP administration.

From the above results, it was found that CHP enhanced muscle function by suppressing the expressions of factors related to aging and muscle atrophy and promoting the expressions of anti-aging and antioxidant genes including mitochondrial function.

### [Example 4]

### Measurement of citrate synthase activity in muscle

Citrate synthase is an enzyme that catalyzes citrate synthesis in the first step of the TCA cycle in mitochondria, and the activity level of this enzyme is used as a biomarker reflecting the amount of mitochondria in muscles (Vigelso et al., The relationship between skeletal muscle mitochondrial citrate synthase activity and whole body oxygen uptake adaptations in response to exercise training, Int J Physiol Pathophysiol Pharmacol, 2014.). In order to measure citrate synthase activity in muscles, proteins were extracted from TA muscles and reacted with oxaloacetate and acetyl-CoA at 37°C to generate CoA-SH. Subsequently, the absorbance was measured at 412 nm by using the chromogenic reagent 5,5'-dithiose (2-nitrobenzoic acid), and the value was corrected for the total protein concentration.

As a result of the experiment, as shown in FIG. 5, it was confirmed that the citrate synthase activity of TA muscle significantly increased by about 12% compared to the control group due to CHP. This result means that the increases in the expressions of genes related to mitochondrial biogenesis and function were reflected in the actual quantitative increase of mitochondria.

### [Example 5]

### Measurement of gene expression changes associated with myocardial atrophy

As a result of CHP reducing skeletal muscle aging and muscle atrophy and enhancing muscle function, changes in cardiac atrophy indices caused by aging were also investigated. As a result of measuring the expressions of *atrogin-1* and *murf-1* genes, which are known as risk factors for cardiac atrophy, by using the same method as in Example 3, it was confirmed that the expressions of the two genes were significantly reduced by CHP.

From the above results, it was found that CHP very effectively enhances muscle function by reducing risk factors for muscle atrophy in skeletal muscle as well as cardiac muscle, and inhibiting muscle aging by increasing antioxidant and mitochondrial activity.

### Statistical Analysis

The statistical significance of the data in FIGS. 1, 2A to 2C, 3A to 3D, 4, 5 and 6 was analyzed by the t-test statistical method. *p<0.05, **p<0.01, ***p<0.001.

### [Example 6]

### Confirmation of CHP effect in DMD animal model

### 6-1. Animal models and CHP treatment

C57BL/10ScSn (BL10) and C57BL/10ScSn-Dmdmdx (mdx) mice were used in this study. Mice were housed at 22°C and allowed free access to food and water. After one week of adaptation, mice were divided into three groups: CHP-treated mdx, water-treated mdx (control) and water-treated BL10 (control). Oral gavage was administered three times a week.

For the prevention protocol, mice were administered with CHP or water (control) at a dose of 20 mg/kg from 3 to 20 weeks of age. For the treatment protocol, mice were administered with CHP or water (control) at a dose of 35 mg/kg from 7 to 22 weeks of age. At the end of the study, mice were euthanized, and tissues were collected. For biochemical analysis, tissues were collected, rapidly frozen and stored at -80°C.

### 6-2. Grip strength test

Grip strength tests were performed at week 10 in the prevention protocol and week 16 in the treatment protocol, respectively, as follows. Grip strength of each mouse on each limb was measured on a pull-down grid assembly connected to a grip dynamometer (Columbus Instruments). Each individual mouse was pulled along a straight line parallel to the grid until the grip broke, providing maximum force (grams). This was repeated three times with a 5-minute interval between each measurement.

As a result, as confirmed in FIGS. 7A and 7B, the mdx mouse groups treated with water in the prevention protocol and the treatment protocol showed a significant decrease in limb muscle strength, but the mdx mouse group treated with CHP showed a significant increase in grip strength.

### 6-3. Hanging Test

The hanging test was performed at week 15 of the treatment protocol as follows. Mice were acclimated to the testing room 30 minutes before the experiment. The animals were placed on a wire grid and were allowed to grasp the grid with their limbs. The grid was then inverted such that the animals were hung upside down, and the time the mice could stay on the grid was measured. The maximum test length was 2 minutes 30 seconds (150 s). The latency to fall was measured five times for each mouse with 10-minute intervals between trials.

As a result, as confirmed in FIG. 8, the average hanging time of the mdx mouse group treated with water significantly decreased, but the average hanging time of the mdx mouse group treated with CHP significantly increased.

The above results confirmed that CHP has the effect of increasing limb muscle strength and exercise endurance.

### [Example 7]

### Contractile and force-generating effects of CHP

### 7-1. Evaluation of muscle strength ex vivo

Force production in limb muscles was tested *ex vivo* in extensor digitorum longus (EDL) and soleus muscles isolated from mice treated with CHP for 17 weeks.

Muscle mechanical measurements were evaluated according to the method described in the literature [N. Zanou et al., "Role of TRPC1 channel in skeletal muscle function," Am. J. Physiol. Cell Physiol., vol. 298, no. 1, Jan. 2010] with minor modifications. All analyses were performed in a blinded manner. BL10, mdx and CHP-treated mdx mice were euthanized by cervical dislocation. EDL and soleus muscles were rapidly dissected and immersed in a 10 mL horizontal chamber containing continuously oxygenated Krebs solution (25°C, pH 7.4) consisting of 135.5 mM NaCl, 5.9 mM KCl, 1 mM MgCl₂, 2 mM CaCl₂, 11.6 mM sodium HEPES and 11.5 mM glucose. The muscle was strapped between a dual-mode lever arm and a fixed hook, and stimulation was delivered via platinum electrodes (1500A Intact Muscle Test System, Aurora Scientific Inc., Canada) running parallel to the muscle. Resting muscle length (L0) was carefully calibrated for maximal isometric force by using 125 Hz maximal fusion tetani. The force-frequency relationship was determined by sequentially stimulating the muscle at stimulation streams of 25, 50, 75, 100, 125 and 150 Hz of 300 ms duration, with 1 minute of rest between each contraction. Normalized muscle force (mN/mm²) was expressed as the cross-sectional area (CSA) obtained by dividing the muscle blot weight (mg) by the length and considering the fiber length as 0.5 L0 for the EDL and 1 for the soleus muscle. Data from each experiment were analyzed by using Aurora's DMA software (Aurora Scientific Inc., 2002; Solwood Enterprises, Inc., 2002) and Microsoft Excel.

### 7-2. Eccentric contraction of EDL muscle ex vivo

Eccentric contractions were performed as described in the literature [N. Zanou, Y. Iwata, O. Schakman, J. Lebacq, S. Wakabayashi, and P. Gailly, "Essential role of TRPV2 ion channels in the sensitivity of dystrophic muscle to eccentric contractions," FEBS Lett., vol. 583, no. 22, pp. 3600-3604, Nov. 2009]. Briefly, the EDL muscle underwent a series of seven eccentric contractions consisting of a 1-mm stretch applied 160 ms after stimulus onset, followed by a 500-ms tonic twitch that lasted up to 250 ms after stimulus onset (with a 10-second interval between two consecutive tetani). Isometric force was measured for each tetanus immediately before stretch onset, and the percentage force decline relative to the first tetanus was calculated.

When tested for concentric contractions, as confirmed in FIGS. 9a to 9f, the muscles of the mdx mice showed overall lower force production and a downward shift in the force-frequency relationship compared to BL10 mice. The performance of EDL and soleus muscles was improved by CHP, with a partial recovery of maximal contractile force. In fact, this is consistent with the preservation of muscle strength observed *in vivo*. These results suggest that CHP treatment maintains force production.

### [Example 8]

### Ca²⁺ release/absorption recovery effect of CHP

There is increasing evidence that sarcolemma damage in DMD muscle fibers as a result of DAPC instability also contributes to the disruption of Ca²⁺ homeostasis. The Ca²⁺ influx and release of muscle fibers is impaired in DMD patients, and the mdx model reports a similar phenotype. In order to evaluate the effect of CHP on Ca²⁺ dysregulation, flexor digitorum brevis (FDB) muscles were isolated from CHP-treated mice at the end of the prevention protocol as well as from healthy control and mdx control mice. Ca²⁺ release from the sarcoplasmic reticulum (SR) was monitored by using the cytosolic Ca²⁺ indicator Fluo-4/AM in isolated fibers treated *ex vivo* with caffeine to stimulate Ca²⁺ release. The experimental procedures are described in more detail below.

FDB muscle fibers were loaded with the cytosolic Ca²⁺ indicator Fluo-4/AM (5 µM; Invitrogen, Basel, Switzerland) dissolved in Krebs Ca²⁺ solution [mM unit: NaCl 135.5, MgCl₂ 1.2, KCl 5.9, glucose 11.5, HEPES 11.5, CaCl₂ 1.8 (pH 7.3)] for 20 minutes in an incubator and then rinsed twice with Krebs solution.

For caffeine stimulation, fibers were washed twice and stored in Krebs Ca²⁺ solution. Fluo-4 fluorescence was monitored by using a confocal microscope system (Zeiss LSM 5 Live, 40x oil immersion lens, excitation wavelength 488 nm and emitted fluorescence recorded between 495 to 525 nm) in a time-lapse acquisition framework. After recording basal fluorescence, fibers were stimulated with a final concentration of 2.5 mM caffeine (O1728-500, Thermofisher Scientific) to induce Ca²⁺ release from the SR. For store-operated Ca²⁺ entry (SOCE) measurements, fibers were washed twice in Ca²⁺-free Krebs solution and then stored in Ca²⁺-free Krebs solution immediately prior to image acquisition.

After recording baseline fluorescence, fibers were stimulated with a final concentration of 1 µM thapsigargin (Tg; T9033, Thermofisher Scientific) to evoke Ca²⁺ release from the SR. 2 mM CaCl₂ was finally used to assess SOCE. Zen software (products/microscopy-software/zenlite/zen-2-lite) was used for acquisition, and data were exported to an Excel file for analysis. The use of the single excitation/release dye fluo-4 necessitates normalization to pre-stimulation values to account for possible differences in dye loading. The amplitude of SR Ca²⁺ storage amount was calculated by subtracting peak fluorescence from baseline. The amplitude of Ca²⁺ transients plateau induced by caffeine stimulation was calculated and expressed as a percentage of the SR Ca²⁺ peak amplitude, indirectly reflecting the contribution to SOCE. Actual SOCE assessed by Tg stimulation was calculated as the difference between the calcium amplitude after addition of 2 mM CaCl₂ and the lowest Ca²⁺ level before addition of CaCl₂. The lowest Ca²⁺ level before CaCl₂ addition was used to estimate the level of Ca²⁺ reuptake (calculated as a percentage of the SR Ca²⁺ peak).

As confirmed in FIG. 10A, the response to caffeine-induced Ca²⁺ release in the SR was impaired in mdx mice because mdx fibers underwent persistent depletion of intracellular Ca²⁺ storage, but was restored by CHP. As confirmed in FIG. 10B, Ca²⁺ uptake via store-operated calcium channels (SOCs) after SR depletion is normally upregulated in mdx as a compensatory mechanism for Ca²⁺ depletion, but CHP normalized Ca²⁺ uptake, indicating that Ca²⁺ handling was properly maintained. In order to directly quantify SOCE, the stimulation of SR Ca²⁺ release was performed by using thapsigargin instead of caffeine, which showed a similar trend of CHP-induced normalization, as confirmed in FIG. 10C and 10D.

### Statistical Analysis

Statistical significance for the data in FIGS. 7A to 7B, 8, 9A to 9F and 10A to 10D was analyzed by one-way analysis of variance (ANOVA) and Dunnett's multiple comparison test. *P<0.05; **P<0.01; ***P<0.001; ****P<0.0001.

## Claims

1. A pharmaceutical composition for enhancing exercise performance, comprising cyclo(his-pro) or a pharmaceutically acceptable salt thereof.

2. The pharmaceutical composition of claim 1, wherein the pharmaceutical composition exhibits any one or more of the following effects of i) to v):
i) increased muscle strength;
ii) increased motor coordination;
iii) increased tolerance to muscle fatigue;
iv) increased balance; and
v) increased muscle energy production.

3. A health functional food composition for enhancing exercise performance, comprising cyclo(his-pro) or a sitologically acceptable salt thereof.

4. The health functional food composition of claim 1, wherein the health functional food composition exhibits any one or more of the following effects of i) to v):
i) increased muscle strength;
ii) increased motor coordination;
iii) increased tolerance to muscle fatigue;
iv) increased balance; and
v) increased muscle energy production.

5. A feed additive for enhancing exercise performance, comprising cyclo(his-pro) or a salt thereof.

6. A pharmaceutical composition for improving muscle function or preventing or treating a muscle disease, comprising cyclo(his-pro) or a pharmaceutically acceptable salt thereof.

7. The pharmaceutical composition of claim 6, wherein the muscle disease is a muscle disease caused by muscle dysfunction, muscle atrophy, muscle loss or muscle degeneration.

8. The pharmaceutical composition of claim 6, wherein the muscle disease is any one or more selected from the group consisting of atony, muscular atrophy, muscular dystrophy, myasthenia, cachexia, sarcopenia, myocardia and acardiotrophy.

9. The pharmaceutical composition of claim 6, wherein the pharmaceutical composition exhibits any one or more of the following effects of i) to v):
i) increased muscle mass;
ii) increased muscle fiber size;
iii) inhibition of muscle loss;
iv) inhibition of muscle atrophy; and
v) increased muscle energy production.

10. A health functional food composition for improving muscle function or preventing or ameliorating a muscle disease, comprising cyclo(his-pro) or a sitologically acceptable salt thereof.

11. The health functional food composition of claim 10, wherein the muscle disease is a muscle disease caused by muscle dysfunction, muscle atrophy, muscle loss or muscle degeneration.

12. The health functional food composition of claim 10, wherein the muscle disease is any one or more selected from the group consisting of atony, muscular atrophy, muscular dystrophy, myasthenia, cachexia, sarcopenia, myocardia and acardiotrophy.

13. The health functional food composition of claim 10, wherein the health functional food composition exhibits any one or more of the following effects of i) to v):
i) increased muscle mass;
ii) increased muscle fiber size;
iii) inhibition of muscle loss;
iv) inhibition of muscle atrophy; and
v) increased muscle energy production.

14. A feed additive for improving muscle function or preventing or ameliorating a muscle disease, comprising cyclo(his-pro) or a salt thereof.

15. A composition for strengthening muscle strength, comprising cyclo(his-pro) or a salt thereof.

16. A method for enhancing exercise performance, comprising administering an effective amount of cyclo(his-pro) or a pharmaceutically or sitologically acceptable salt thereof to a subject in need thereof.

17. A method for improving muscle function or preventing, ameliorating or treating a muscle disease, comprising administering an effective amount of cyclo(his-pro) or a pharmaceutically or sitologically acceptable salt thereof to a subject in need thereof.

18. A method for strengthening muscle strength, comprising administering an effective amount of cyclo(his-pro) or a pharmaceutically or sitologically acceptable salt thereof to a subject in need thereof.

19. Use of cyclo(his-pro) or a pharmaceutically or sitologically acceptable salt thereof for the manufacture of a medicament or health functional food for enhancing exercise performance.

20. Use of cyclo(his-pro) or a pharmaceutically or sitologically acceptable salt thereof for the manufacture of a medicament or health functional food for improving muscle function or preventing, ameliorating or treating a muscle disease.

21. Use of cyclo(his-pro) or a pharmaceutically or sitologically acceptable salt thereof for the manufacture of a medicament or health functional food for strengthening muscle strength.
